# EUROPEAN PATENT SPECIFICATION

(11) **EP 0 685 213 B1**
(45) Date of publication and mention of the grant of the patent: **24.10.2001**
(21) Application number: 95107487.1
(22) Date of filing: 16.05.1995
(51) Int. Cl.: A61F 13/15, A61F 13/20

(54) **Tampon with integral cover**
Tampon mit integrierter Verkleidung
Tampon avec garniture intégrée

(30) Priority: 31.05.1994 US 251769
(43) Date of publication of application: 06.12.1995
(73) Proprietor: KIMBERLY-CLARK WORLDWIDE, INC., Neenah, Wisconsin 54956 (US)
(72) Inventor: Chambers, Leon Eugene, Jr., Cumming, GA 30131 (US); Jackson, David Martin, Roswell, GA 30076 (US); Mann, Walter Bly, Chester, Cheshire CH2 3HQ (GB); Thomas, David Glyn, Flint CH6 5H2, Wales (GB)
(74) Representative: Grünecker, Kinkeldey, Stockmair & Schwanhäusser Anwaltssozietät

(56) References cited:
- WO-A-84/03833
- US-A- 3 683 912
- US-A- 3 784 425
- US-A- 3 976 075
- DATABASE WPI Section Ch, Week 7820 Derwent Publications Ltd., London, GB; Class A96, AN 78-35617A XP002021873 & JP-A-53 036 996 (UNI CHARM KK) , 5 April 1978

## Description

The present invention relates to a process for forming a tampon of the type as defined in the preamble of claim 1.

A process for forming a tampon of this type is disclosed in US-A-5,084,038. The process described in that publication includes a step of pre-forming a strip of a cover material and a ribbon of an absorbent material. Both the pre-formed cover strip as well as the absorbent ribbon are cut independently from each other into segments of predetermined length. Thereafter, one segment of the cover strip is put onto one segment of the absorbent ribbon, and the overlaying materials are transferred to a heating device where they are bonded firmly.

WO 84/03833 describes a process for manufacturing a tampon which includes mixing of thermally meltable fibres with the liquid absorbing fibres at least in the outer layer of the tampon, and thereafter applying heat to the outer layer in a manner to cause the meltable fibres to fuse together in order to form a network which binds the liquid absorbent fibres.

Tampons are a widely used means by women for controlling and absorbing menstrual flow. Typically, the absorbent used in tampons is a combination of cotton and rayon which is compressed into a generally cylindrical shape having a length of approximately 5 centimeters and a diameter of between about 1 and 2 centimeters. The tampons themselves are packages with or without insertion devices depending upon the preference of the end user.

Tampons have been manufactured without any type of cover material. It was found, however, the short cotton fibers were better retained through the addition of a cover material. In addition, the tampons lacking covers were oftentimes difficult to insert. With the use of covers, lubricants and other additives could be employed which would facilitate the insertion process.

A common practice in the manufacture of covered tampons is to overlay a pre-formed cover material in registration with the absorbent core material. Strips of the two layer material are then spirally wound into cylinders with the cover material forming the exposed surface of each of the cylinders. The cylinders are then radially compressed to form the resultant tampons.

A problem with the foregoing design has centered around the proper indexing of the cover material on top of the absorbent core material and subsequently keeping the two layers in registration throughout the remainder of the tampon converting process. In addition, when working with preformed finite length rolls of material, there is the inevitable problem of reduced efficiency as the rolls of cover material are replaced after being depleted in the formation process. As a result, there is a need for an improved method of manufacture of a tampon.

This need is fulfilled by the process of claim 1.

The present invention is directed to a process for forming a personal care product such as a tampon with an integral cover. The process comprises forming a thermally bondable fibrous nonwoven surface web directly onto an exterior surface of a fibrous nonwoven web absorbent core to form a laminate. The surface web or cover material is water-insoluble and is formed by extruding a plurality of molten fibers directly onto the surface of the absorbent core to form a fibrous nonwoven web cover material which is adhered to the absorbent core via the formation process and the latent heat of the fibers. As a result of forming the cover material directly onto the absorbent core, the separate steps of forming a preformed cover material and placing it in direct contact with the absorbent core and maintaining registration are eliminated. Once the cover material has been formed on and adhered to the absorbent core material, a strip of the laminate is cut and rolled into a spirally wound cylinder with the cover web forming an exposed surface on the spirally wound cylinder. The spirally wound cylinder is then radially compressed to densify the laminate into the conventional shape of a tampon. At the same time or directly after the compression step, heat may be applied to the compressed cylinder to heat and partially fuse the fibers of the cover web. The compression results in a tampon having a density of between about 0.4 and about 0.8 grams per cubic centimeter.

To further enhance insertion of the tampon by the end user, a lubricant may be sprayed onto or added to the fibers forming the cover web. The lubricant may be sprayed onto the molten thermoplastic fibers as they are being extruded but before they are deposited onto the top surface of the absorbent core. Alternatively, the lubricant may be sprayed onto the fibrous cover web after the web has been deposited onto the absorbent core material.

In an alternative embodiment, the molten thermoplastic fibers that are deposited onto the absorbent core as a cover material may include a first component having a first melting point and a second component having a second melting point. Such fibers may be formed as, for example, biconstituent fibers or bicomponent fibers. The laminate using these fibers is formed in the same fashion as described above, however, during the heating and fusing step, a temperature is applied which is greater than the melting point temperature of the lower melting component but less than the melting point temperature of the higher melting component thereby causing the first component to melt and bond the fibers together while maintaining the integrity of the second component.

As yet a further embodiment of the present invention, instead of using biconstituent or bicomponent fibers, the cover material may be made from a multiplicity of different fibers, some of which may be used for bonding purposes. The bonding fibers, which will typically have a lower melting point than the other fibers, act to bond the cover material and to bond the cover material to the absorbent core.

As a further refinement of the product and process, the deposition of the cover material onto the surface of the absorbent core may be cycled on and off to intermittently form areas of laminate separated by areas of absorbent core. In the past, the cover material overlapped a majority of the length of the tampon laminate but only the last approximately two to three inches (5 to 7.6 centimeters) of the cover material were actually employed in the lamination and formation of the exterior surface or cover. Consequently, the excess cover material which was spirally wound up and contained within the interior of the tampon was wasted and in some cases presented processing problems. By intermittently depositing the cover material onto the surface of the absorbent core material, significant material savings and processing efficiencies can be achieved. To form the tampon, the same process as described above is used. However, the strip of tampon material must be spirally wound from the non-laminate end of the strip so the laminate end, which has the cover material, forms the complete exterior surface of the cylinder once the winding is complete. The spirally wound cylinder of tampon material is then radially compressed and optionally heated to partially fuse the cover material and increase the density of the resultant tampon.

### BRIEF DESCRIPTION OF THE DRAWINGS

Figure 1 is a schematic side view of the process for making a tampon with an integral cover according to the present invention.

Figure 2 is a cross-sectional side view of the laminate used to make a tampon according to the process of the present invention.

Figure 3 is an end view of the spiral wrapping pattern used to form an, as yet, uncompressed tampon according to the process of the present application.

Figure 3a is an end view of another spiral wrapping pattern used to form an, as yet, uncompressed tampon according to the process of the present invention.

Figure 4 is a side view of a tampon formed in accordance with the process of the present invention.

Figure 5 is a cross-sectional side view of yet another piece of material formed by the process according to the present invention which can be formed into tampons.

Figure 6 depicts a portion of the material shown in Figure 5 being spirally wound into an, as yet, uncompressed tampon according to the present invention.

Figure 7 is a schematic side view of a meltspray process which may be used to form the fibers and resultant integral cover of the material of the present invention. This collective process is referred to as element 46 in the process of Figure 1.

Figure 8 is a cross-sectional side view of a melt spray die assembly as used in the process of Figure 7.

### DETAILED DESCRIPTION OF THE INVENTION

The present invention is directed to personal care absorbent products such as diapers, training pants, incontinence garments, sanitary napkins, tampons, bandages and the like. More particularly, the present invention is directed to a process for forming a personal care product such as a tampon.

A conventional tampon is traditionally made from a strip of absorbent material such as rayon and/or cotton which is formed into a rectangular strip usually having dimensions of 50 mm by 400 mm and a basis weight of between about 150 gsm and about 250 gsm. To one surface of the absorbent core material there is placed a second layer of preformed cover material such as a powder-bonded carded web containing powdered adhesive. The two layers are spirally wound with the cover material positioned to the exterior. Once the two layers have been wound into a spiral, the spiral is radially compressed into a self-sustaining cylindrical shape with a substantially hemispherical frontal portion or tip, i.e., a "bullet" shape, and then heated to melt and fuse the adhesive bonding powder of the cover material thereby forming the exterior surface or cover of the finished tampon product.

The present invention is distinguishable from the preceding process in that it involves the extrusion of molten thermoplastic polymer into fibers which are randomly deposited directly onto one surface of the absorbent core while the molten fibers are still within a semi-molten state, thereby forming a fibrous nonwoven web cover material which is attached directly to one surface of the absorbent core as a result of the fiber formation and deposition process. The molten fibers adhere at their crossover points to themselves and the fibers have sufficient tackiness to at least partially adhere themselves to the surface of the absorbent core onto which they are deposited. In so doing, the tampon cover material can be applied in line thereby alleviating the need to preform a separate cover and index it onto the absorbent core. As a result, the simplicity of the converting process can be increased and the cost of the overall process can be reduced. Once the fibrous nonwoven web cover has been applied to the absorbent core, the composite or laminate can be spirally wound, radially compressed and heat set in the same fashion as conventional tampons.

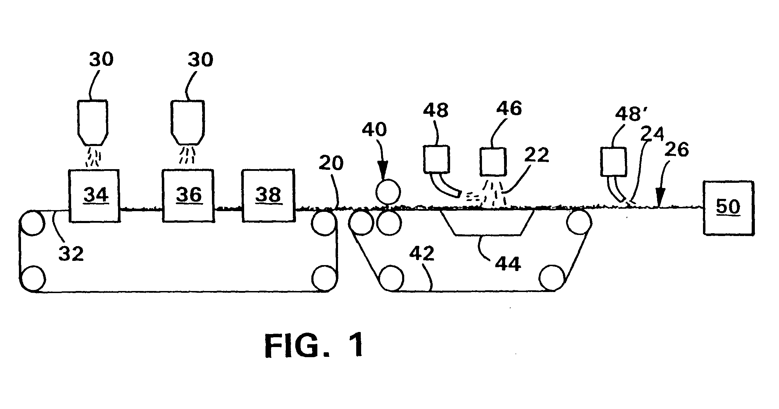

Referring to Figure 1 there is shown a schematic of the process of the present invention. The first step in the present invention is to form an absorbent core material 20. The specific composition of the absorbent core material 20 is not critical provided the material is capable of absorbing viscous fluids such as menses. In Figure 1 the absorbent core 20 is made from a carded web of short staple fibers. Such carding processes are well known and need not be described herein in detail. The absorbent core material 20 is made from a combination of rayon and cotton fibers which are blended, carded, plied up, calendered and slit to form a fleece material. The supplies of fibers are fed into a hopper or hoppers 30 where the fibers are separated by a picker. From the hoppers 30, the fibers are then directed into a first carding machine 34 and a second carding machine 36. After exiting the second carding machine 36, the rayon and cotton fibers are laid down on a first moving foraminous wire 32 as a nonwoven web 20 and the web is in turn passed into a drum-lapper 38 which produces a higher basis weight, multi-ply material by plying several layers of material 20 onto itself. From there, a portion of the multi-ply absorbent core material is fed into a slitter and embosser 40 where the carded web 20 is slit and then densified into widths of approximately 10.2 centimeters (4 inches). From the slitter and embosser 40, the absorbent core material 20 is directed over a second foraminous wire 42 which has a vacuum box 44 underneath the forming wire 42 to pull the absorbent core material 20 down onto its surface. As the absorbent core material 20 passes over the vacuum box 44, a supply of molten thermoplastic fibers is directed down onto the surface of the absorbent core material 20 positioned away from the foraminous wire 42. The molten thermoplastic fibers 22 are expelled from an extrusion die 46 which may be a part of any conventional thermoplastic fiber forming nonwoven process such as a meltblown, meltspray, spunbond or solution spun process. Such forming processes are well known and need not to be described herein in detail. See for example Appel, et al., U.S. Patent 4,340,563, Dorschner et al., U.S. Patent 3,692,618; Kinney, U.S. Patent Numbers 3,338,992 and 3,341,394; Levy, U.S. Patent Number 3,276,944; Peterson, U.S. Patent 3,502,538; Hartman, U.S. Patent 3,502,763; and Dobo et al., U.S. Patent Number 3,542,615. The fibers formed from such processes are essentially continuous in that the fiber lengths are longer than conventional staple fibers. Oftentimes the ratio of the fiber lengths to fiber widths will approach infinity.

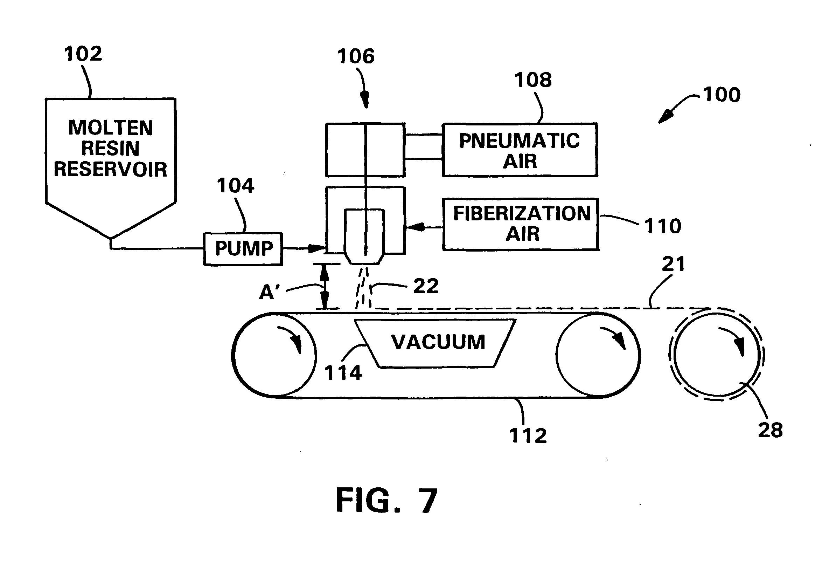

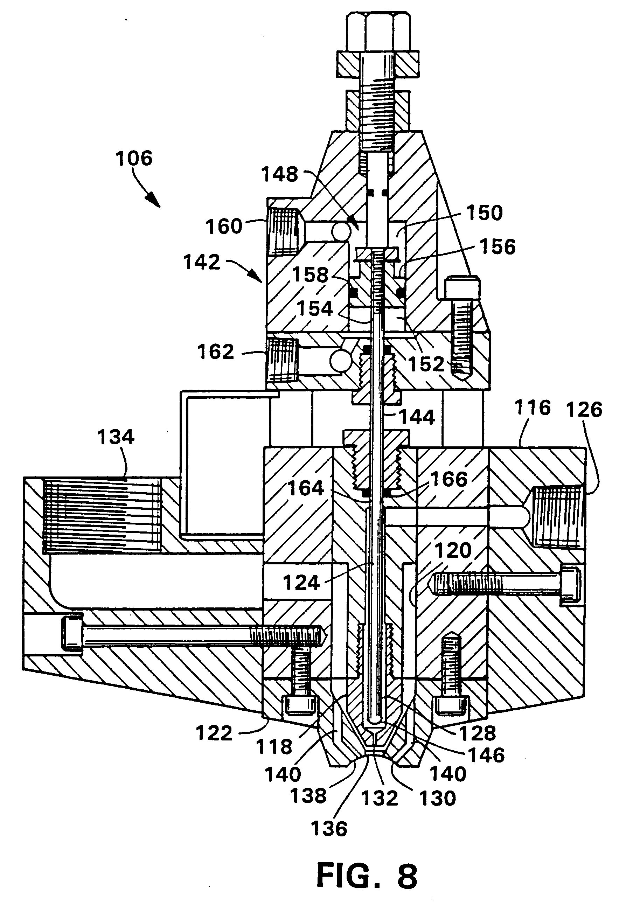

In meltspraying, an apparatus is used to either continuously or intermittently form and spray fibers onto a forming surface or another substrate. Referring to Figures 7 and 8, the apparatus 100 may be used as the fiber deposition apparatus 46 in Figure 1 and includes a reservoir 102 for melting the heat bondable polymer resin and maintaining the resin in the molten state. Typically heat bondable thermoplastic resins melt at temperatures in the range of 149 to 260°C. Therefore, the reservoir must be able to maintain resin temperatures to at least within this range. A pump 104 pumps the molten resin from the reservoir 102 to one or more fiber forming dies generally indicated at 106. The apparatus 100 includes a source of pressurized air 108 for operating on/off control means and a source of fiberization fluid 110 to fiberize the molten resin as discussed below. Note that a single die 106 or a multiple die assembly may be used to form fibers and nonwoven cover materials according to the present invention.

The fibers 22 emanating from the die 106 are collected on a receiver assembly such as a continuous wire forming belt 112 in the form of a web 21. The receiver assembly can include means 114 for producing a vacuum beneath the receiving portion of the belt 112 to effectively hold the web 21 to the belt surface and affect the density of the resultant nonwoven web 21. The receiving surface of the belt 112 is spaced at a predetermined distance A' from the die 106.

Referring to Figure 8, the fiber forming die 106 has a main housing 116 for receiving a die assembly including a resin nozzle 118 which is fitted within an air forming chamber 120 and capped with an air plate 122. The resin nozzle 118 is in turn fitted with a retractable plunger assembly 124 (a part of the on/off control means) which will permit interruption of the resin flow and cleaning of the nozzle orifice. The die 116 is adapted to receive supplies of both air and molten resin. The air is separately used to operate the retractable plunger assembly 124 and to draw and attenuate the molten resin into fibers.

The molten resin first enters the main housing 116 of the die 106 through the resin inlet port 126 which leads into the interior of the nozzle 118 located within the die 106. The nozzle 118 contains a resin chamber or main flow body 128 which houses and surrounds the hydraulically actuated plunger assembly 124. Consequently, the resin inlet port 126 and main flow body 128 are in fluid communication with one another. As the molten resin enters the main flow body 128, it fills and pressurizes the chamber. The molten resin is then released from the chamber through a resin fluid capillary 130 to form fibers via a resin outlet orifice 132 located within the air plate assembly 122. Initially, the plunger assembly 124 is seated against the base of the resin outlet orifice 132 thereby preventing release of the molten resin. When the plunger 124 is retracted and therefore unseated from the resin outlet orifice 132, the resin is then permitted to escape from the main flow body 128 and thus begin the formation of the fibers 22.

To fiberize and attenuate the resin exiting the resin outlet orifice 132, fiberization/attenuation air or other fluid is used to surround and attenuate the resin into fibers 22. Consequently, the die means 106 is equipped with primary and, if desired, secondary fiberization means for drawing and attenuating the fibers 22. Air or another fluid fiberization source enters the die 106 through a fluid inlet port 134. As can be seen from Figure 8, the fluid inlet port 134 is in fluid communication with the air forming chamber 120 which is formed by the space between the interior of the main die housing 116/air plate 128 of the die 106 and the exterior of the nozzle 118. The air forming chamber 120 surrounds at least the lower portion of the nozzle 118 and extends into the air plate assembly 122 where it terminates in an annular fluid outlet port 136. The fluid outlet port 136 typically has a diameter ranging from 3.0 to 5.0 mm. It is this fluid outlet port 136 which forms the primary means for attenuating and fiberizing the fibers 22. As the fluid outlet port 136 is reduced in diameter, the fiberization/attenuation air is increased in velocity causing the fibers 22 to be attenuated more severely.

To further attenuate and fiberize the molten fibers 22, a secondary fiberization means may also be used. Referring to Figure 8, the air plate assembly 122 may be fitted with secondary fluid outlet ports 138 spaced radially and axially outward from the first or primary fluid outlet port 136 to create a plurality of secondary fluid streams which impinge upon and further fiberize the molten resin into the fibers. The secondary fluid outlet ports 138 are in fluid communication with the air supply 110 in Figure 7 via fluid channels 140 which connect the secondary fluid outlet ports 138 with the air forming chamber 120. Alternatively, the secondary fluid outlet ports 138 may be connected to an independent pressurized fluid source so the type and/or pressure emanating therefrom may be controlled independently of the primary fiberization fluid.

The on/off control means includes a pneumatic fixture generally indicated at 142 which is connected to and therefore forms a part of the main die housing 116. Extending from the pneumatic fixture 142 into the main flow body 128 is a plunger assembly or reciprocating stem 144 having a distal tip 146 located above the resin flow capillary 130. The stem 144 has an unseated condition wherein the tip 146 is retracted into the main flow body 128 and is spaced from the capillary 130 and a seated condition wherein the stem 144 is reciprocated to seat the tip 146 against the capillary 130. By seating the tip 146, a hydrostatic pressure is created in the capillary 130 which dislodges any debris located therein which would restrict the flow of molten resin from the resin flow outlet 132.

The pneumatic fixture 142 includes a pneumatic chamber 148, including an upper chamber 150 and lower chamber 152 shown in Figure 8. The stem 144 includes an end portion 154 extending into the pneumatic chamber 148. The end portion 154 of the stem 144 has a piston 156 mounted thereon and fitted with seals 158 to contact the walls of the chamber 148 to form the upper and lower chambers 150 and 152, respectively. The chamber 148 includes a pair of hydraulic fluid ports 160, 162 opening into the pneumatic chamber 148 for supplying varying fluid pressure on each side of the piston 156 to reciprocate the piston 156 within the pneumatic chamber 148 thereby reciprocating the tip 146 between the seated (off) and unseated (on) conditions. The main flow body 128 includes a stem port 164 with the stem 144 extending through the stem port 164. The die 106 includes a high temperature resistant dynamic seal 166 for allowing sliding engagement while perfecting a seal between the stem 144 and the stem port 164 to prevent the passage of molten resin through the port 164. The seal 166 can be U-shaped in cross-section so as to expand into the space between the stem 144 and the wall of the port 164 when pressure is applied by the incoming polymer resin. Thus, the seal 166 provides a separation between the molten material and the outside environment under molten material pressure conditions. The seal mechanism should provide a positive seal at temperatures up to 350°C.

Operation of the on/off mechanism involves selectively pressurizing either the upper chamber 150 or the lower chamber 152. To turn the mechanism on and start the flow of molten resin from resin outlet orifice 132, the pressure from upper chamber 150 is relieved through fluid port 160 and pressurized air is fed into lower chamber 152 via fluid port 162. As a result of the pressure imbalance on either side of the piston 156, the piston 156 moves further into the upper chamber 150 unseating the tip 146 of the stem 144 from the capillary 130 and thereby allowing the release of the molten resin from the main flow body 128 through resin outlet orifice 132. To turn off the mechanism and interrupt the flow of molten resin the above procedure is reversed. Namely, the pressure from the lower chamber 152 is relieved and the pressure in the upper chamber 150 is increased, again causing a pressure imbalance which forces the tip 146 of stem 144 to seat against the capillary 130 and cut off the flow of molten resin. Additionally, this action will create a sufficient hydrostatic pressure within capillary 130 to dislodge any debris contained therein. See U.S. Patent Number 5,160,746 to Dodge et al.

Suitable polymers from which to form the fibers 22 include polymers which yield fibers which are thermally bondable to one another when subjected to heat and/or pressure. Examples of such polymers include, but are not limited to, polyethylene, polypropylene, polybutylene, polyethylene terephathalate, polybutylene terephathalate, ethylene vinyl acetate, nylon, etc. In addition, blends and copolymers of such polymers or fibers may also be used. Of particular use with the present invention are biconstituent and bicomponent fibers such as, for example, polypropylene/polybutylene biconstituent fibers. Biconstituent or multiconstituent fibers have two or more polymers randomly mixed along the length of the fiber generally in a discontinuous manner. Bicomponent or multicomponent fibers contain two or more polymers which are generally present in fairly distinct regions of the fiber which run generally continuously parallel to the longitudinal axis of the fiber. Both types of fibers normally contain a first component having a first melting point and a second component having a second melting point which is higher than the first. By heating the fibers to a temperature above the first melting point but below the second, bonding of the fibers can be effected. The melting point of a polymer is determined at the peak value of the melting endotherm as measured by differential scanning calorimetry.

Returning to Figure 1, as the molten fibers 22 exit the die tip 46, they may be treated with a lubricant 24 from a spray nozzle 48. The purpose of the lubricant 24 is to coat the molten fibers 22 so that upon ultimate formation of the tampon, the external surface of the tampon will have a lower degree of friction than without the lubricant and therefore will be more easily inserted into the end user. Examples of such lubricants include such compounds as cosmetic emollients, emulsions, etc. and specifically Cetiol 1414-E (myreth-3-myristate) from Henkel Corporation of Cincinnati, Ohio which is described in detail in U.S. Patent Number 4,377,167 to Kaczmarzyk et al. and U.S. Patent Number 4,300,561 to Kaczmarzyk et al.

As the molten thermoplastic fibers 22 contact and adhere to the top surface of the absorbent core material 20 they are brought into more intimate contact with the surface of the absorbent core material due to the application of a vacuum via the vacuum box 44 shown in Figure 1. After the molten fibers 22 have been deposited upon the absorbent core material 20 a laminate 26 is formed which includes the absorbent core material 20 and the cover material 21 formed from the extruded thermoplastic fibers 22.

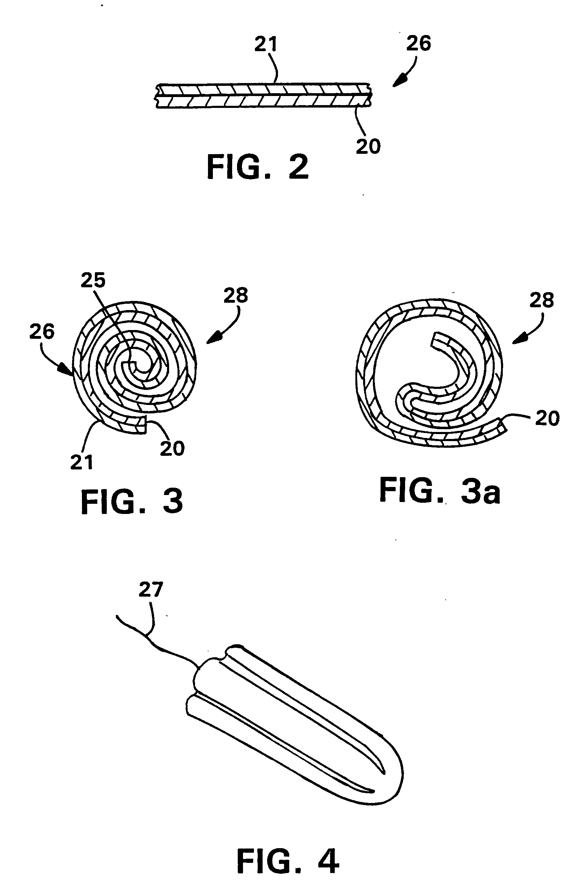

Once the laminate 26 has been formed, it is cut into 5.1 centimeter by 38.1 centimeter (2 inch x 15 inch) strips by a slitter and pull roll assembly 49 and is then spirally wound into cylinders 28 such as are shown in Figure 3 with the cover material 21 positioned on the exposed or exterior surface of the cylinder 28. At this point the remainder of the process is depicted by element 50 in Figure 1. This portion of the tampon formation process uses conventional forming techniques such as are taught in U.S. Patent Numbers 5,084,038 to Sheldon et al.; 4,498,218 to Friese; 3,422,496 to Wolff et al.; 4,951,368 to Heinen; 4,081,884 to Johst et al. and 4,109,354 to Rone.

As shown in Figure 3, the laminate 26 is spirally wound into a tampon cylinder 28 starting at one end 25 of the laminate 26. It is also possible to spirally wind the laminate 26 starting at a point midway between the ends of the laminate 26. See Figure 3a. In so doing, at least a portion of the laminate 26 will be folded back over on itself. Thus "spirally winding", "spirally wound", etc. should be construed to mean any folding pattern which ultimately results in the cover material 21 forming the exterior surface of the resultant tampon 29.

As part of the process depicted by element 50, the tampon cylinders 28 are radially compressed by mechanical compressors (not shown) which include a plurality of dies which reciprocate relative to one another so as to form a mold cavity. The tampon cylinders 28 are placed within the mold cavities and the dies are moved toward one another to radially compress the cylinders 28. The compression causes the cylinders 28 to be reduced in diameter to a fraction of their original diameter. In the formation of tampons, the compressed material, which is known as a pledget, will usually have a diameter of less than about 12 millimeters (one half of an inch) and a density between about 0.4 and about 0.8 grams per cubic centimeter (g/cc). The compressed cylinders 28 are heated to a sufficiently high temperature to partially melt and fuse the fibers 22 of the cover material 21. Care should be taken to not overly heat and fuse the fibers 22 to a point where the porosity of the cover material 21 is adversely affected and the resultant tampon cannot adequately absorb and retain the menses. Generally the pledgets will be heated to a temperature of between about 95°C and about 155°C for a period of approximately 20 to 40 seconds. When using biconstituent or bicomponent fibers as all or a part of the cover material 21, the degree of heating, which may be done with or without pressure, and the length of time should be sufficient to cause bonding of the lower melting point component while maintaining the integrity of the higher melting point component of the fibers.

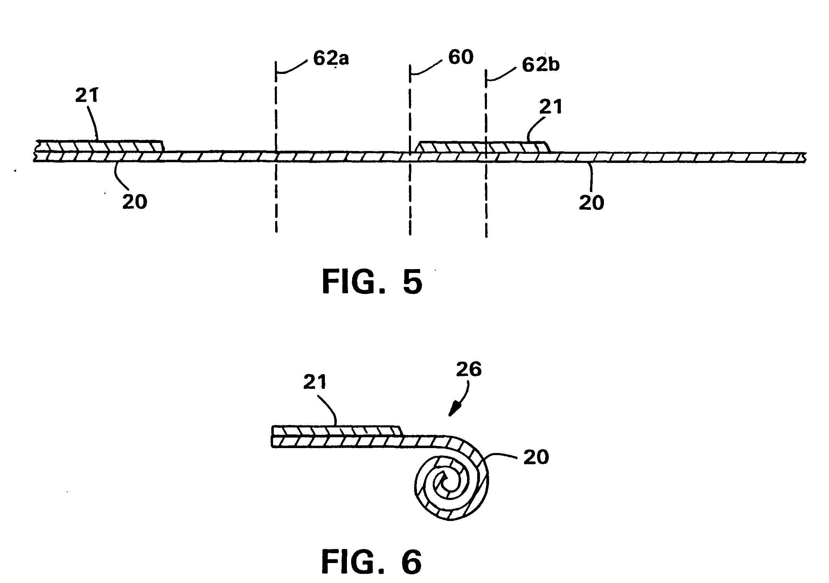

The resultant tampon 29, will typically have a bullet-shaped form such as is shown in Figure 4 with the cover material 21 forming the exterior surface of the resultant tampon 29. If desired, a removal string 27 may be incorporated into the tampon during the formation process. The string is typically 100 percent cotton thread which is treated with an anti-wicking agent and may or may not be dyed. The addition of such removal strings are well known and taught, as for example, in U.S. Patent Numbers 5,006,116 to Alikhan et al.; 3,595,236 to Corrigan and 3,724,465 to Duchane, all of which are incorporated herein by reference in their entirety.

If desired, the foregoing process can be modified without departing from the scope of the present invention. For example, as opposed to applying the lubricant 24 to the molten fibers 22 during their formation, the spray applicator 48 may be moved downstream from the extrusion equipment 46 to a position such as is shown by 48' in Figure 1. In this position, the lubricant is applied to the fibers 22 of the cover material 21 after they have been laid down onto the surface of the absorbent core 20 and have already formed a fibrous nonwoven web laminate 26.

As shown in Figure 1, the molten thermoplastic fibers 22 are applied in a continuous manner along the entire length of the absorbent core material 20. However, from a functional standpoint, the cover material 21 needs only to be applied to the surface of the absorbent core material 20 in a sufficient length to completely cover the exposed exterior surface and circumference of the resultant tampon. As a result, if so desired, the extruding apparatus 46 may be cycled on and off to create a material such as is shown in cross-section in Figure 5 with areas of cover material 21 separated by areas of absorbent core material 20 which are essentially devoid of fibers 22/cover material 21. By "essentially devoid" it is meant that the particular section of the strip chosen for evaluation contains no more than sixty percent by weight of cover web material as compared to the weight of the same size section of cover material which forms the exterior surface/cover of the finished tampon. In addition, that section which is essentially devoid of cover material must not form the entire exposed surface of the resultant tampon. Once the strip of material shown in Figure 5 has been formed, it is then cut along dashed line 60 or dashed lined 62a and 62b to form a strip of laminate material 26 such as is shown in Figure 6.

Referring to Figure 6, the cover layer 21 is only applied to the last approximately 5.1 centimeters (2 inches) of the overall composite 26. As the tampon is spirally wound, it is wound in such a manner so that the last portion of the laminate 26 to be wound into the spiral 28 contains the cover material 21 on the exposed surface along a sufficient portion of its length so as to completely encompass the exterior surface of the cylinder 28. Then, upon compression and heating, the resultant tampon 29 can be formed without unduly wasting cover material within the interior portions of the spirally wound laminate.

Having thus described the materials and process of the present invention, a series of examples were prepared to demonstrate the attributes and advantages of the present invention.

### EXAMPLES

The first step in the formation of the tampons according to the present invention was to create the absorbent core material or fleece using a blend of bleached cotton fibers and 1.5 denier, 42 millimeter long rayon staple fibers. The cotton fibers were cotton comber grade BP 1018 from Edward Hall of Whaley Bridge, England. The rayon fibers were Svenska Swelan 983 standard rayon fibers with a glycerol finish and were obtained from Svenska Rayon of Valberg, Sweden. The blend of fibers typically included about 80 to 95 weight percent rayon fibers and about 5 to 20 weight percent cotton fibers based upon the total weight of the absorbent core web. The fibers were uniformly blended and carded into a nonwoven web having a basis weight of approximately 200 grams per square meter. The web of absorbent core material as formed had an initial width of 150 centimeters but was subsequently plied upon itself using a drum-lapper to yield a final material which was 15 plies thick. From the drum-lapper, a strip of material approximately 10 to 11 centimeters wide was cut and fed through a pair of spring-loaded calendar rolls to densify the material to a density of approximately 0.05 grams per cubic centimeter. The cover material was then formed directly onto one surface of the absorbent core material.

To form the cover material, a meltspray unit of the type described above with respect to Figures 7 and 8 was used. Polymer for the cover material was supplied to the spray head using a Meltex MX 4060 Grid Melter from Nordson Corporation of Norcross, Georgia. The spray head unit was a Nordson CF204 Stainless Steel Spray Head with four polymer nozzles from the same manufacturer. The spray head was positioned at a distance (A') which ranged from 15 to 40 centimeters from the top surface of the absorbent core material. Polymer output and absorbent core material line speed varied with each sample as indicated below. In some of the examples a lubricant was also added to the fibers.

After the cover material was deposited onto the absorbent core material, the resultant laminate was slit into 5.5 centimeter wide strips which were in turn cut into 40 centimeter lengths. As the strips were being cut into 40 centimeter lengths, the strips of laminate were also pulled, thereby causing a partial tearing of the laminate. This in turn created loose strands of cover fibers which further aided in the heat-sealing of the overlapping portions of the cover material.

Each of the strips of laminate material was spirally wound into cylinders with the cover material forming the exterior surface. The cylinders were then radially compressed and heated using conventional tampon forming equipment such as Nova tampon converting equipment manufactured by K. Fassbind-Ludwig. The tampons were heated while under compression to a temperature which was generally about 10 degrees Celsius higher than the melting point of the specific polymer within the cover material which was being used for bonding. The bonding time was approximately 20 to 40 seconds. Densities for the resultant tampons ranged between about 0.4 and about 0.8 grams per cubic centimeter.

### EXAMPLE I

In Example I the fibrous nonwoven web absorbent core was a 110 millimeter wide, 230 gram per square meter (gsm) fleece material made from 85 weight percent rayon fibers and 15 weight percent cotton fibers. Onto the surface of the fleece there was deposited a cover material made from a thermally bondable fibrous nonwoven surface web using Himont HH-442H polypropylene (PF-015) polymer from Himont, U.S.A. Incorporated. Throughput of the polymer was adjusted so as to deposit a web of approximately 10 gsm onto the fleece. As the molten polypropylene fibers were being deposited onto the fleece a 50% solution of Cetiol 1414-E lubricant in water was sprayed into the molten fibers at an angle of 90 degrees to the polymer spray direction. The target application rate was 10 to 12 percent solids by weight based upon the total weight of the cover material.

### Example II

In Example II the fleece and cover material which were prepared were the same as in Example I with the exception that polymer throughput was increased thereby causing the cover material to have a basis weight of 15 gsm. The Cetiol level was the same as in Example I.

### Example III

In Examples I and II the polymer spray head was positioned between the calendar and embossing rolls and the resultant laminates had hard spots. In Example III, the spray head was positioned downstream of the embossing rolls. The same fleece and cover material were used as in Examples I and II. Lubricant was added to the cover material polymer stream as it was being deposited onto the surface of the fleece in the same fashion as in Examples I and II. Throughput rates of the molten polypropylene polymer and the carding machine were adjusted to create laminates with cover material weights of 10, 15 and 20 gsm.

Various samples of the laminates from the preceding examples were converted into tampons and subjectively evaluated. Based upon appearance and integrity, it was determined that the 20 gsm surface webs yielded better tampon covers as there was less twisting and pilling of the fibers making up the tampon covers.

### Example IV

In Example IV, four samples were prepared with the variables consisting of the polymers being used in the cover material and whether or not a lubricant was added to the cover material. The fleece was the same as that used in the previous examples and the lubricant was the same 50 percent Cetiol lubricant solution in warm water, approximately 50°C.

For sample IVa, a 20 gsm web was formed directly onto the absorbent core material using a polymer mixture with a weight percent blend of 40 percent Duraflex DP 8911 polybutylene and 60 percent Himont Profax PF-015 polypropylene. Weight percents for both polymers were based upon the total weight of polymers in the cover material. The Cetiol lubricant solution was applied at an approximate add-on of 6 percent solids by weight based upon the total weight of the cover material. Sample IVb was produced in the same manner as sample IVa except for the deletion of the lubricant spray.

For sample IVc, the only change relative to sample IVa was the polymer mixture of the surface web fibers which formed the cover material. The cover material of sample IVc was approximately 20 gsm and contained 20 weight percent polybutylene and 80 weight percent polypropylene. The web also contained the same amount of lubricant as sample IVa. Sample IVd was the same as sample IVc except for the deletion of the lubricant in the surface web.

Tampons were then made as before from pieces of the four sample laminates according to the present invention. Subjectively these tampons felt softer and smoother to the touch than the previous samples though all samples were acceptable.

Having thus described the invention in detail, it should be apparent that various modifications and changes can be made to the present invention without the departing from the scope of the following claims.

## Claims

1. A process for forming a tampon comprising:
forming a laminate (26) of a thermally bondable nonwoven cover material (21) and an absorbent core material (20)
rolling a strip of said laminate (26) into a spirally wound cylinder (28) with said nonwoven cover material (21) forming an exposed exterior surface of said spirally wound cylinder (28), and
radially compressing said cylinder (28)
**characterised in that** said laminate (26) is formed by extruding a plurality of fibres (22) onto a surface of said absorbent core material (20) to form a fibrous nonwoven cover material (21) which is adhered to the absorbent core (20) via the formation process and the latent heat of the fibres, and said cylinder (28) is compressed to a density of between about 0.4 and about 0.8 grams per cubic centimetre.

2. The process of claim 1 which further includes the step of heating said cylinder (28) to at least partially fuse said exposed exterior surface of said cylinder (28).

3. The process of claim 1 or 2 which further includes the step of applying a lubricant (24) to said nonwoven cover material (21).

4. The process of claim 3 wherein said lubricant (24) is applied to said nonwoven cover material (21) after said nonwoven cover material (21) is formed onto said absorbent core material (20).

5. The process of claim 3 which further includes the step of applying said lubricant (24) to said nonwoven cover material (21) during said forming step and before said nonwoven cover material (21) is formed onto a surface of said absorbent core material (20).

6. The process of any one of claims 1 to 5 wherein said nonwoven cover material (21) includes a plurality of multi-constituent or multi-component fibres with a first component having a first melting point and a second component have a second melting point.

7. The process of claim 6 which further includes the step of heating said laminate (26) to a temperature greater than said first melting point and less than said second melting point to at least partially fuse said exposed exterior surface of said cylinder (28).

8. The process of claim 6 or 7 wherein said fibres contain biconstituent fibres.

9. The process of claim 8 wherein said biconstituent fibres contain polypropylene and polybutylene.

10. The process of any one of claims 1 to 9 **characterised by**
intermittently forming said thermally bondable fibrous nonwoven cover material (21) onto said surface of said absorbent core material (20) to form a tampon material having laminated areas formed of said nonwoven cover material (21) and said absorbent core material (20) separated by non-laminated areas, said non-laminate areas being essentially devoid of said nonwoven cover material (21), and
cutting said tampon material into a plurality of strips each of which contains a laminated area of a non-laminated area.

## Patentansprüche

1. Verfahren zum Herstellen eines Tampons wobei:
ein Laminat (26) aus einem thermisch bindbaren, nicht gewebten Abdeckmaterial (21) und einem absorbierenden Kernmaterial (20) hergestellt wird,
ein Streifen des Laminats (26) in einen spiralig gewickelten Zylinder (28) gerollt wird, wobei das nicht gewebte Abdeckmaterial (21) eine freiliegende, äußere Oberfläche des spiralig gewickelten Zylinders (28) bildet, und
der Zylinder (28) radial komprimiert wird,
**dadurch gekennzeichnet, dass** das Laminat (26) durch Extrudieren einer Vielzahl von Fasern (22) auf eine Oberfläche des absorbierenden Kemmaterials (20) hergestellt wird, um ein Abdeckmaterial (21) aus nicht gewebten Fasern zu bilden, das am absorbierenden Kem (20) durch den Herstellungsprozess und die latente Wärme der Fasern anhaftet, und dass der Zylinder (28) auf eine Dichte zwischen etwa 0,4 und etwa 0,8 g/cm³ komprimiert wird.

2. Verfahren nach Anspruch 1, das ferner den Verfahrensschritt des Erwärmens des Zylinders (28) umfasst, um die freiliegende äußere Oberfläche des Zylinders (28) mindestens teilweise zu verschweißen.

3. Verfahren nach Anspruch 1 oder 2, das femer den Verfahrensschritt des Aufbringens eines Gleitmittels (24) auf das nicht gewebte Abdeckmaterial (21) enthält.

4. Verfahren nach Anspruch 3, wobei das Gleitmittel (24) auf das nicht gewebte Abdeckmaterial (21) aufgebracht wird, nachdem das nicht gewebte Abdeckmaterial (21) auf dem absorbierenden Kernmaterial (20) ausgebildet wurde.

5. Verfahren nach Anspruch 3, das ferner enthält den Verfahrensschritt des Aufbringens des Gleitmittels (24) auf das nicht gewebte Abdeckmaterial (21) während des Herstellungsschrittes und bevor das nicht gewebte Abdeckmaterial (21) auf einer Oberfläche des absorbierenden Kernmaterials (20) ausgebildet wird.

6. Verfahren nach einem der Ansprüche 1 bis 5, wobei das nicht gewebte Abdeckmaterial (21) eine Vielzahl von Mehrkomponenten-Fasem enthält, wobei eine erste Komponente einen ersten Schmelzpunkt und eine zweite Komponente einen zweiten Schmelzpunkt aufweist.

7. Verfahren nach Anspruch 6, das ferner umfasst den Verfahrensschritt des Erwärmens des Laminats (26) auf eine Temperatur, die größer als der erste Schmelzpunkt und geringer als der zweite Schmelzpunkt ist, um zumindest teilweise die freiliegende äußere Oberfläche des Zylinders (28) zu verschmelzen.

8. Verfahren nach Anspruch 6 oder 7, wobei die Fasern Bikomponentenfaser enthalten.

9. Verfahren nach Anspruch 8, wobei die Bikomponentenfasern Polypropylen und Polybutylen enthalten.

10. Verfahren nach einem der Ansprüche 1 bis 9, **gekennzeichnet durch** ein unterbrochenes Ausbilden des nicht gewebten Abdeckmaterials (21) aus thermisch bindbaren Fasermaterial auf der Oberfläche des absorbierenden Kemmaterials (20), um ein Tamponmaterial zu bilden, das laminierte Flächenbereiche aufweist, die aus dem nicht gewebten Abdeckmaterial (21) und dem absorbierenden Kernmaterial (20) hergestellt sind, getrennt **durch** nicht laminierte Bereiche, wobei die nicht laminierten Bereich im Wesentlichen frei von dem nicht gewebten Abdeckmaterial (21) sind und Schneiden des Tamponmaterials in eine Vielzahl von Streifen, wobei jeder einen laminierten Bereich und einen nicht laminierten Bereich enthält.

## Revendications

1. Procédé de formation d'un tampon comprenant :
la formation d'un stratifié (26) d'une matière de garniture (21) non-tissée pouvant être fixée thermiquement et d'une matière de noyau absorbant (20) le roulement d'une bande dudit stratifié (26) en un cylindre enroulé en spirale (28) avec ladite matière de garniture non-tissée (21) qui forme une surface extérieure exposée dudit cylindre enroulé en spirale (28), et
la compression radiale dudit cylindre (28)
**caractérisé en ce que** ledit stratifié (26) est formé par extrusion d'une pluralité de fibres (22) sur une surface de ladite matière de noyau absorbant (20) pour former une matière de garniture non-tissée fibreuse (21) qui est collée au noyau absorbant (20) au moyen du procédé de formation et de la chaleur latente des fibres, et ledit cylindre (28) est comprimé à une densité de l'ordre de 0,4 environ à 0,8 grammes environ par centimètre cube.

2. Procédé selon la revendication 1, comprenant en outre l'étape de chauffage dudit cylindre (28) pour fondre au moins partiellement ladite surface extérieure exposée dudit cylindre (28).

3. Procédé selon la revendication 1 ou 2, comprenant en outre l'étape d'application d'une matière lubrifiante (24) à ladite matière de garniture non-tissée (21).

4. Procédé selon la revendication 3, dans lequel ladite matière lubrifiante (24) est appliquée à ladite matière de garniture non-tissée (21) après que ladite matière de garniture non-tissée (21) est formée sur ladite matière de noyau absorbant (20).

5. Procédé selon la revendication 3, comprenant en outre l'étape d'application de ladite matière lubrifiante (24) à ladite matière de garniture non-tissée (21) pendant ladite étape de formation et avant que ladite matière de garniture non-tissée (21) soit formée sur une surface de ladite matière de noyau absorbant (20).

6. Procédé selon l'une quelconque des revendications 1 à 5, dans lequel ladite matière de garniture non-tissée (21) comprend une pluralité de fibres à plusieurs constituants ou à plusieurs éléments avec un premier élément ayant un premier point de fusion et un deuxième élément ayant un deuxième point de fusion.

7. Procédé selon la revendication 6, comprenant en outre l'étape de chauffage dudit stratifié (26) à une température supérieure audit premier point de fusion et inférieure audit deuxième point de fusion pour fondre au moins partiellement ladite surface extérieure exposée dudit cylindre (28).

8. Procédé selon la revendication 6 ou 7, dans lequel lesdites fibres contiennent des fibres à deux constituants.

9. Procédé selon la revendication 8, dans lequel lesdites fibres à deux constituants contiennent du polypropylène et du polybutylène.

10. Procédé selon l'une quelconque des revendications 1 à 9, **caractérisé par**
la formation par intervalles de ladite matière de garniture non-tissée (21) fibreuse, pouvant être fixée thermiquement sur ladite surface de ladite matière de noyau absorbant (20) pour former une matière de tampon ayant des zones feuilletées formées de ladite matière de garniture non-tissée (21) et de ladite matière de noyau absorbant (20) séparée par des zones non-feuilletées, lesdites zones non-feuilletées étant essentiellement exemptes de ladite matière de garniture non-tissée (21),
le découpage de ladite matière de tampon en une pluralité de bandes contenant chacune une zone feuilletée d'une zone non-feuilletée.
